# EUROPÄISCHE PATENTSCHRIFT

(11) **EP 1 105 388 B1**
(45) Veröffentlichungstag und Bekanntmachung des Hinweises auf die Patenterteilung: **02.05.2003**
(21) Anmeldenummer: 99942792.5
(22) Anmeldetag: 20.07.1999
(51) Int. Cl.: C07D 403/06, A61K 31/4184

(54) **N-SUBSTITUIERTE AZABICYCLOHEPTAN-DERIVATE, IHRE HERSTELLUNG UND VERWENDUNG**
N-SUBSTITUTED AZABICYCLOHEPTANE DERIVATIVES, PRODUCTION AND USE THEREOF
DERIVES A SUBSTITUTION N D'AZABICYCLOHEPTANE, PREPARATION ET UTILISATION DESDITS DERIVES

(30) Priorität: 12.08.1998 DE 19836404
(43) Veröffentlichungstag der Anmeldung: 13.06.2001
(73) Patentinhaber: BASF AKTIENGESELLSCHAFT, 67056 Ludwigshafen (DE)
(72) Erfinder: STEINER, Gerd, D-67281 Kirchheim (DE); HÖGER, Thomas, D-68535 Edingen-Neckarhausen (DE); UNGER, Liliane, D-67065 Ludwigshafen (DE); TESCHENDORF, Hans-Jürgen, D-67373 Dudenhofen (DE); JUCHELKA, Frieder, D-69181 Leimen (DE)
(74) Vertreter: Grünecker, Kinkeldey, Stockmair & Schwanhäusser Anwaltssozietät
(86) Internationale Anmeldenummer: EP9905166
(87) Internationale Veröffentlichungsnummer: WO00009499

(56) Entgegenhaltungen:
- WO-A-94/00431
- WO-A-94/00458
- WO-A-95/15312
- WO-A-96/04245
- WO-A-96/04272
- US-A- 4 254 127

## Beschreibung

Die Erfindung betrifft neue N-substituierte Azabicydoheptan-Derivate, deren Herstellung und Verwendung zur Bekämpfung von Krankheiten.

Exo-6-Phenyl-3-azabicyclo[3.2.0]heptan-Derivate besitzen interessante Eigenschaften als potentielle Neuroleptika (WO 94/00458, WO 95/15312). Hierbei spielen die beobachteten hohen Affinitäten zu D₄- und 5-HT₂-Rezeptoren eine besondere Rolle.

Die interessanteste bekannte Substanz aus obigen Verbindungsklassen mit hoher D₄/5-HT_{2A}-Affinität und guter Selektivität versus D₂ ist (+)-(1S, 5R, 6S)-exo-3-[2-[6-(4-Fluorphenyl)-3-azabicyclo-[3.2.0]heptan-3-yl]-ethyl]-1H, 3H-chinazolin-2, 4-dion (= Substanz A), die ein potentielles Neuroleptikum darstellt. Die Dosierung der Substanz A ist allerdings nach oben begrenzt durch auftretende Verlängerungen des QT-Intervalls am Herz-EKG.

In der WO 96/04272 werden N-substituierte 3-Azabtcycto[3.2.0]heptan-Derivate und deren Eigenschaften als Neuroleptika beschrieben.

Es wurden nun Substanzen mit besseren Eigenschaften gefunden.

Gegenstand der Erfindung sind N-substituierte 3-Azabicyclo-[3.2.0]heptan-Derivate der Formel I 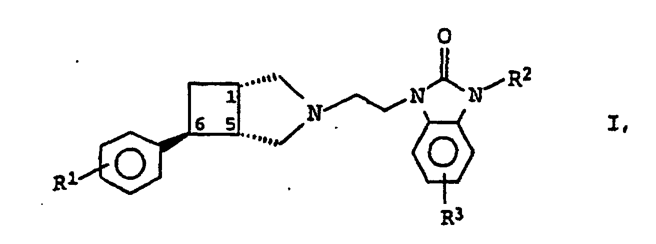 worin
- R¹: Fluor oder Chlor bedeutet,
- R²: C₁-C₃-Alkyl oder Cyctopropyl bedeutet, und
- R³: Wasserstoff, Fluor oder Chlor ist,
und deren Salze mit physiologisch vertraglichen Säuren.

Bevorzugt sind die Verbindungen, in denen
- R¹: Chlor, vorzugsweise in der p-Stellung,
- R²: Methyl oder Cyclopropyl und
- R³: Wasserstoff
bedeuten.

Die folgenden Verbindungen sind als besonders bevorzugt zu nennen:
(+)-(1S,5R,6S)-exo-1-[2-[6-(4-Chlor-phenyl)-3-azabicyclo-[3.2.0]heptan-3-yl]-ethyl]-3-methyl-2H-1,3-dihydro-benzimidazol-2-on,
(+)-(1S,5R,6S)-exo-1-[2-[6-(4-Chlor-phenyl)-3-azabicyclo[3.2.0]heptan-3-yl]-ethyl]-3-ethyl-2H-1,3-dihydro-benzimidazol-2-on und
(+)-(1S,5R,6S)-exo-1-[2-[6-(4-Fluor-phenyl)-3-azabicyclo-[3.2.0]-heptan-3-yl]-ethyl]-3-cyclopropyl-2H-1,3-dihydro-benzimidazol-2-on.

Die erfindungsgemäßen Verbindungen der Formel I lassen sich herstellen, indem man eine Verbindung der Formel II 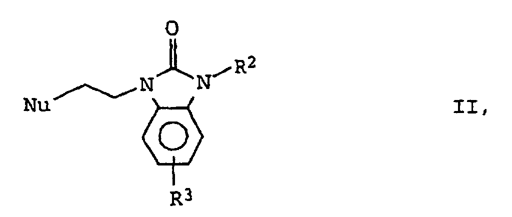 in der R² und R³ die oben angegebenen Bedeutungen haben und Nu eine nukleofuge Abgangsgruppe darstellt, mit einem 3-Azabicyclo-[3.2.0]heptan-Derivat der Formel III als (+)-(1S,5R,exo-6S)-Enantiomeres 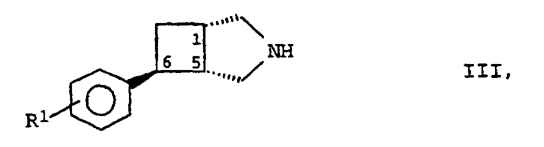 worin R¹ die oben angegebene Bedeutung hat, umsetzt und die so erhaltene Verbindung gegebenenfalls in das Säureadditionssalz einer physiologisch verträglichen Säure überführt.

Als nukleofuge Abgangsgruppe für Nu kommen vorzugsweise Halogenatome, insbesondere Brom oder Chlor, in Betracht.
Die Umsetzung erfolgt zweckmäßig in Gegenwart einer inerten Base, wie Triethylamin oder Kaliumcarbonat, als säurebindendes Mittel in einem inerten Lösungsmittel, wie einem cyclischen gesättigten Ether, insbesondere Tetrahydrofuran oder Dioxan, oder einem Benzolkohlenwasserstoff, wie Toluol oder Xylol.

Die Umsetzung erfolgt in der Regel bei Temperaturen von 20 bis 150°C, insbesondere von 80 bis 140°C, und ist im allgemeinen innerhalb von 1 bis 10 Stunden beendet.

Die erfindungsgemäßen Verbindungen der Formel I können entweder durch Umkristallisation aus den üblichen organischen Lösungsmitteln, bevorzugt aus einem niederen Alkohol, wie Ethanol, umkristallisiert oder durch Säulenchromatographie gereinigt werden.

Die freien 3-Azabicyclo[3.2.0]heptan-Derivate der Formel I können in üblicher Weise in das Säureadditionssalz einer pharmakologisch verträglichen Säure überführt werden, vorzugsweise durch Versetzen einer Lösung mit einem Äquivalent der entsprechenden Säure. Pharmazeutisch verträgliche Säuren sind beispielsweise Salzsäure, Phosphorsäure, Schwefelsäure, Methansulfonsäure, Amidosulfonsäure, Maleinsäure, Fumarsäure, Oxalsäure, Weinsäure oder Zitronensäure.

Die erfindungsgemäßen Verbindungen weisen wertvolle pharmakologische Eigenschaften auf. Sie können als Neuroleptika (insbesondere atypische), Antidepressiva, Sedativa, Hypnotika, ZNS-Protektiva oder Mittel zur Behandlung der Cocain-Abhängigkeit Verwendung finden. Mehrere der genannten Wirkungsqualitäten können kombiniert bei einer erfindungsgemäßen Verbindung auftreten.

Die Substanzen sind insbesondere durch eine sehr hohe und selektive Affinität zum Dopamin D₄- und Serotonin 2A-Rezeptor charakterisiert.

Die am Modell des Meerschweinchen-Papillarmuskels gemessenen Verlängerungen des QT-Intervalls sind vernachlässigbar gering. Deshalb sind die neuen Substanzen auch in hohen Dosierungen gut verträglich.

Die Erfindung betrifft dementsprechend auch ein therapeutisches Mittel, gekennzeichnet durch einen Gehalt an einer Verbindung der Formel I oder deren pharmakologisch verträgliches Säureadditionssalz als Wirkstoff neben üblichen Träger- und Verdünnungsmitteln, sowie die Verwendung der neuen Verbindungen bei der Bekämpfung von Krankheiten.
Die erfindungsgemäßen Verbindungen können in üblicher Weise oral oder parenteral, intravenös oder intramuskulär verabfolgt werden.

Die Dosierung hängt vom Alter, Zustand und Gewicht des Patienten sowie von der Applikationsart ab. In der Regel beträgt die tägliche Wirkstoffdosis zwischen etwa 1 und 100 mg/kg Körpergewicht bei oraler Gabe und zwischen 0,1 und 10 mg/kg Körpergewicht bei parenteraler Gabe.

Die neuen Verbindungen können in gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et. al: Pharmazeutische Technologie, Thieme-Verlag, Stuttgart, 1978).
Die so erhaltenen Applikationsformen enthalten den Wirkstoff normalerweise in einer Menge von 1 bis 99 Gew.-%.

Die als Ausgangsstoffe für die Synthese der erfindungsgemäßen Verbindungen benötigten Substanzen der Formel II und III sind bekannt (WO 94/00458; Heterocycles 40 (1), 319-330 (1995); J. Heterocyclic Chem. 18, 85-89 (1981) oder lassen sich gemäß den in der Literatur beschriebenen Herstellmethoden aus analogen Ausgangsmaterialien synthetisieren.

Die folgenden Beispiele dienen zur Erläuterung der Erfindung:

### A Herstellung der Ausgangsmaterialien

a) 1-(α-Phenylvinyl)-2H-1,3-dihydro-benzimidazol-2-on
   21,6 g (200 mM) o-Phenylendiamin und 37 ml (214 mM) Ethylbenzoylacetat wurden in 75 ml 4-tert.-Butyltoluol unter Zugabe einer Spatelspitze p-Toluolsulfonsäure 1 h bei 200°C Badtemperatur unter Stickstoff am Wasserabscheider zum Rückfluß erhitzt und das freigesetzte Wasser abgetrennt. Nach dem Abkühlen versetzte man das Reaktionsgemisch mit 80 ml Acetonitril, rührte im Eisbad nach und saugte die Festkörper unter Nachwaschen mit kaltem Acetonitril ab. Man isolierte 39,5 g (84 %) Produkt, Smp. 167-169°C.
   In analoger Weise läßt sich 1-(α-Phenylvinyl)-6-chlor-2H-1,3-dihydro-benzimidazol-2-on herstellen (Ausgangsmaterial: 4-Chlor-1,2-diaminobenzol).
b) 1-(α-Phenylvinyl)-3-methyl-2H-1,3-dihydro-benzimidazol-2-on
   3,5 g (14,8 mM) 1-(α-Phenylvinyl)-2H-1,3-dihydrobenzimidazol-2-on in 70 ml Toluol wurden mit 40 ml 10 %iger Natronlauge, 0,2 g Benzyl-triethyl-ammoniumchlorid und 1,76 ml (18,5 mM) Dimethylsulfat versetzt und 2 h bei 60°C nachgerührt. Anschließend engte man die Toluol-Phase ein und isolierte 3,65 g (99 %) Produkt als Öl in genügender Reinheit für die weitere Umsetzung.
   In analoger Weise wurden hergestellt: 1-(α-Phenylvinyl)-3-ethyl-2H-1,3-dihydro-benzimidazol-2-on (Alkylierungsmittel: Diethylsulfat) und 1-(α-Phenylvinyl)-3-n-propyl-2H-1,3-dihydro-benzimidazol-2-on (Alkylierungsmittel 1-Brompropan), 1-(α-Phenylvinyl)-3-methyl-6-chlor-2H-1,3-dihydro-benzimidazol-2-on (Ausgangsmaterial: 1-(α-Phenylvinyl)-6-chlor-2H-1,3-dihydro-benzimidazol-2-on) sowie 1-(2-Chlorethyl-3-methyl-5-chlor-2H-1,3-dihydro-benzimidazol-2-on (Ausgangsmaterial: 1-(2-Chlorethyl-5-chlor-2H-1,3-dihydro-benzimidazol-2-on).
c) 1-Methyl-2H-1,3-dihydro-benzimidazol-2-on
   3,65 g (14,6 mM) 1-(α-Phenylvinyl)-3-methyl-2H-1,3-dihydro-benzimidazol-2-on wurden in 30 ml Ethanol gelöst, mit 60 ml 10 %iger Salzsäure sowie mit 10 ml konzentrierter Salzsäure versetzt und 2 h bei 80°C gerührt. Anschließend wurde der Ansatz eingeengt und zur verbleibenden wäßrigen Lösung Eis zugesetzt. Die ausgefallenen Festkörper wurden unter Kühlung im Eisbad ausgerührt, abgesaugt und mit Wasser nachgewaschen. Man isolierte 1,7 g (79 %) Produkt.
   Analog wurden hergestellt: 1-(2-Chlorethyl)-5-chlor-2H-1,3-dihydro-benzimidazol-2-on (Ausgangsmaterial: 1-(2-Chlorethyl)-3-(α-phenylvinyl)-5-chlor-2H-1,3-dihydro-benzimidazol-2-on) und 1-Methyl-5-chlor-2H-1,3-dihydro-benzimidazol-2-on (Ausgangsmaterial: 1-(α-Phenylvinyl)-3-methyl-6-chlor-2H-1,3-dihydro-benzimidazol-2-on).
d) 1-(2-Chlorethyl)-3-methyl-2H-1,3-dihydro-benzimidazol-2-on
   1,7 g (11,5 mM) 1-Methyl-2H-1,3-dihydro-benzimidazol-2-on in 40 ml Acetonitril wurden mit 1,6 g (11,5 mM) fein gepulvertem Kaliumcarbonat und 2,9 ml (35 mM) 1-Brom-2-chlor-ethan versetzt und 14 h am Rückfluß gekocht. Nach dem Abkühlen saugte man die Festkörper ab, wusch mit Acetonitril nach und engte anschließend das Filtrat ein. Man isolierte 2,3 g (95 %) Produkt als Öl, das langsam kristallisierte, Smp. 87-89°C.
   Analog wurden hergestellt: 1-(2-Chlorethyl)-3-(α-phenylvinyl)-5-chlor-2H-1,3-dihydro-benzimidazol-2-on (Ausgangsmaterial: 1-(α-Phenylvinyl)-6-chlor-2H-1,3-dihydrobenzimidazol-2-on) und 1-(2-Chlorethyl)-3-methyl-6-chlor-2H-1,3-dihydro-benzimidazol-2-on (Ausgangsmaterial: 1-Methyl-5-chlor-2H-1,3-dihydro-benzimidazol-2-on).
e) (+)-(1S,5R,6S)-exo-6-(4-Chlor-phenyl)-3-azabicyclo[3.2.0]heptan
   Die Isolierung des (+)-Enantiomeren wurde gemäß der Vorschrift in Heterocycles 40 (1), 326 (1995) vorgenommen.
f) 1-(α-Phenylvinyl)-3-cyclopropyl-2H-1,3-dihydro-benzimidazol-2-on
   Zu 10,0 g (42,4 mM) 1-(α-Phenylvinyl)-2H-1,3-dihydrobenzimidazol-2-on in 80 ml Dimethylformamid wurde portionsweise unter gutem Rühren 1,35 g (45 mM) Natriumhydrid (80-prozentig) zugegeben und die Reaktionsmischung noch 2 h nachgerührt. Anschließend fügte man 8,0 ml (100 mM) Cyclopropylbromid hinzu und überführte das Reaktionsgemisch in einen 0,3-1-Rührautoklaven, der dann 10 h auf 200°C aufgeheizt wurde. Nach dem Abkühlen engte man am Rotationsverdampfer ein und verteilte den Rückstand zwischen Methylenchlorid und Wasser unter Ansäuern mit 10-prozentiger Salzsäure. Die wäßrige Phase extrahierte man nochmals mit Methylenchlorid nach. Nach Trocknen und Einengen der organischen Phase isolierte man 12,4 g Rohprodukt, das durch Säulenchromatographie (Kieselgel, Laufmittel Methylenchlorid) gereinigt wurde. Ausbeute 3,6 g (31 %) in genügender Reinheit.

### B Herstellung der Endprodukte

### Beispiel 1

### (+)-(1S,5R,6S)-exo-1-[2-[6-(4-Chlor-phenyl)-3-azabicyclo[3.2.0]heptan-3-yl]-ethyl]-3-methyl-2H-1,3-dihydro-benzimidazol-2-on Tartrat x 2H₂O

2,5 g (12,1 mM) (+)-(1S,5R,6S)-exo-6-(4-Chlor-phenyl)-3-azabicyclo[3.2.0]heptan in 60 ml Xylol wurden mit 2,55 g (12,1 mM) 1-(2-Chlor-ethyl)-3-methyl-2H-1,3-dihydro-benzimidazol-2-on und 1,7 g (12,1 mM) fein pulverisiertem Kaliumcarbonat versetzt und 20 h am Rückfluß gekocht. Anschließend engte man am Rotationsverdampfer ein, verteilte den Rückstand zwischen Wasser und Methyl-tert.-butylether bei pH 10. Die wäßrige Phase extrahierte man nochmals mit Methyl-tert.butylether und engte darauf die vereinigten organischen Phasen ein. Das Rohprodukt reinigte man durch Säulenchromatographie (Kieselgel, Laufmittel Methylenchlorid/Methanol 97/3. Man isolierte 3,3 g (71 %) Produkt als Öl, das in 200 ml Ether gelöst und mit 1,4 g (9,3 mM) Weinsäure, gelöst in Ethanol, in das Tartrat (Smp. 107 bis 109°C) übergeführt wurde. [α]_{D} = + 55,4° (EtOH)

| Elementaranalyse C₂₂H₂₄N₃OCl x C₄H₆O₆ x 2H₂O | | | | | |
|---|---|---|---|---|---|
| Berechnet | C 54,97 | H 6,03 | N 7,39 | O 25,34 | Cl 6,24 |
| Gefunden | C 54,9 | H 5,8 | N 7,1 | O 25,5 | Cl 6,2 |

In analoger Weise wurden hergestellt:
2. (+)-(1S,5R,6S)-exo-1-[2-[6-(4-Chlor-phenyl)-3-azabicyclo[3.2.0]heptan-3-yl]-ethyl]-3-ethyl-2H-1,3-dihydro-benzimidazol-2-on x HCl, Smp. 174 bis 176°C, [α]_{D} = + 67,7° (EtOH)
3. (+)-(1S,5R,6S)-exo-1-[2-[6-(4-Chlor-phenyl)-3-azabicyclo[3.2.0]heptan-3-yl]-ethyl]-3-n-propyl-2H-1,3-dihydro-benzimidazol-2-on x HCl, Smp. 178 bis 180°C, [α]_{D} = + 66,4° (EtOH)
4. (+)-(1S,5R,6S)-exo-1-[2-[6-(4-Chlor-phenyl)-3-azabicyclo[3.2.0]heptan-3-yl]-ethyl]-3-methyl-5-chlor-2H-1,3-dihydrobenzimidazol-2-on x HCl, Smp. 101 bis 103°C, [α]_{D} = + 92,3° (EtOH)
5. (+)-(1S,5R,6S)-exo-1-[2-[6-(4-Chlor-phenyl)-3-azabicyclo[3.2.0]heptan-3-yl]-ethyl]-3-methyl-6-chlor-2H-1,3-dihydrobenzimidazol-2-on x HCl, Smp. 230 bis 232°C, [α]_{D} = + 77,2° (EtOH)
6. (+)-(1S,5R,6S)-exo-1-[2-[6-(4-Chlor-phenyl)-3-azabicyclo[3.2.0]heptan-3-yl]-ethyl]-3-cyclopropyl-2H-1,3-dihydrobenzimidazol-2-on x HCl, Smp. 109 bis 111°C, [α]_{D} = + 73,5° (EtOH)
7. (+)-(1S,5R,6S)-exo-1-[2-[6-(4-Fluor-phenyl)-3-azabicyclo[3.2.0]heptan-3-yl]-ethyl]-3-cyclopropyl-2H-1,3-dihydrobenzimidazol-2-on x HCl, Smp. 121 bis 123°C, [α]_{D} = + 64,5° (EtOH)

## Patentansprüche

1. N-substituierte 3-Azabicyclo[3.2.0]heptan-Derivate der Formel I 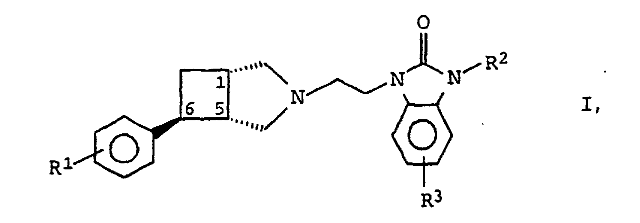 worin
R¹ Fluor oder Chlor bedeutet,
R² C₁-C₃-Alkyl oder Cyclopropyl bedeutet, und
R³ Wasserstoff, Fluor oder Chlor ist,
und deren Salze mit physiologisch verträglichen Säuren.

2. Verbindungen der Formel I gemäß Anspruch 1 zur Verwendung bei der Bekämpfung von Krankheiten.

## Claims

1. N-substituted 3-azabicyclo[3.2.0]heptane derivatives of the formula I 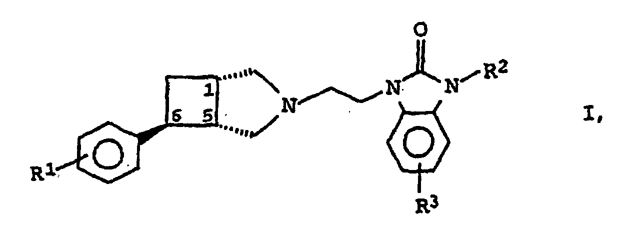 wherein
R¹ denotes fluorine or chlorine,
R² denotes C₁-C₃-alkyl or cyclopropyl, and
R³ is hydrogen, fluorine or chlorine,
and the salts thereof with physiologically tolerated acids.

2. Compounds of the formula I according to claim 1 for use in combating diseases.

## Revendications

1. Dérivés du 3-azabicyclo[3.2.0]heptane N-substitués de formule I : 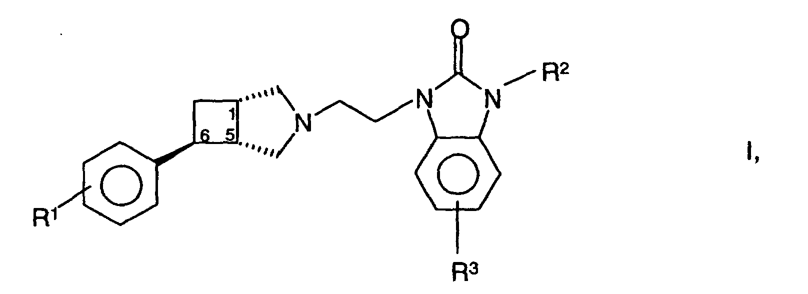 où
R¹ représente le fluor ou le chlore,
R² représente alkyle en C₁-C₃ ou cyclopropyle, et
R³ est l'hydrogène, le fluor ou le chlore,
et leurs sels avec des acides physiologiquement acceptables.

2. Composés de formule I selon la revendication 1 destinés à être utilisés dans la lutte contre des maladies.
